# EUROPEAN PATENT SPECIFICATION

(11) **EP 4 021 902 B1**
(45) Date of publication and mention of the grant of the patent: **20.03.2024**
(21) Application number: 20764911.2
(22) Date of filing: 24.08.2020
(51) Int. Cl.: C07D 403/04, A61K 31/513, A61P 35/00

(54) **CRYSTALLINE FORMS OF A CD73 INHIBITOR**
KRISTALLINE FORMEN EINES CD73-INHIBITORS
FORMES CRISTALLINES D'UN INHIBITEUR DE CD73

(30) Priority: 29.08.2019 EP 19382733
(43) Date of publication of application: 06.07.2022
(73) Proprietor: Eli Lilly and Company, Indianapolis, IN 46285 (US)
(72) Inventor: GARCIA-CERRADA, Susana Maria, Indianapolis, Indiana 46206-6288 (US); LU, Yu, Indianapolis, Indiana 46206-6288 (US); REMICK, David Michael, Indianapolis, Indiana 46206-6288 (US)
(74) Representative: Smith, Andrew George
(86) International application number: PCT/US2020/047645
(87) International publication number: WO 2021/041319

(56) References cited:
- WO-A1-2017/120508
- WO-A1-2019/168744
- RAHILA RAHIMOVA ET AL: "Identification of allosteric inhibitors of the ecto-5'-nucleotidase (CD73) targeting the dimer interface", PLOS COMPUTATIONAL BIOLOGY, vol. 14, no. 1, 29 January 2018 (2018-01-29), page e1005943, XP055584046, DOI: 10.1371/journal.pcbi.1005943
- MINO R CAIRA ED - MONTCHAMP JEAN-LUC: "Crystalline Polymorphism of Organic Compounds", TOPICS IN CURRENT CHEMISTRY; [TOPICS IN CURRENT CHEMISTRY], SPRINGER, BERLIN, DE, vol. 198, 1 January 1998 (1998-01-01), pages 163-208, XP008166276, ISSN: 0340-1022, DOI: 10.1007/3-540-69178-2_5 [retrieved on 1999-02-26]

## Description

The present invention relates to crystalline forms of 5-[5-[2-isopropylcyclopropyl] -6-methyl-pyridazin-3-yl]-1H-pyrimidine-2,4-dione, and pharmaceutical compositions comprising them, which inhibit the activity of CD73, and are useful for treating cancer.

CD73, also known as 5'-nucleotidase or ecto-5'nucleotidase (EC 3.1.3.5), is an enzyme that converts 5' mononucleotides to nucleosides. CD73 is expressed in many tissues, and is upregulated in cancerous tissues, and the CD73 pathway promotes tumor growth by limiting antitumor T cell immunity via adenosine receptor signaling (Zhang B, Cancer Research 2010; 70: 6407-6411; Antonioli L, et al., Drug Discovery Today 2017; 22: 1686-1696). CD73-deficient mice have increased antitumor immunity, and are resistant to experimental metastasis (Stagg J, et al., Cancer Research 2011; 71: 2892-2900). Extracellular adenosine generated by tumor CD73 accumulates in the tumor microenvironment, impairs antitumor T cell immunity (Zhang B; Antonioli L), and is linked to immune escape by tumors, proliferation, migration, neovascularization, metastasis and chemoresistance of tumor cells (Inoue Y, et al., Oncotarget 2017; 8: 8738-8751). Elevated CD73 expression has also been reported to be associated with increased immune suppression (Jin D, et al., Cancer Res. 70: 2245-2255 (2010)).

Thus, the CD73 pathway exerts an immune-suppressive effect, and it has been suggested that blocking the CD73 pathway can be useful in treating cancer (Allard D, et al., Immunotherapy 2016; 8: 145-163). CD73 inhibitors are in clinical trials for the treatment of cancer (Hay CM, et al., Oncoimmunol. 2016; 5(8): e1208875; Allard D). Rahimova et al., Plos Computational Biology 2018, vol. 14, pages 1-23 and WO2017/120508 disclose CD73 inhibitors.

U.S. application no. 16/481,146, entitled, "CD73 Inhibitors," has been published as WO2019/168744 and discloses certain CD73 inhibitor molecules, including the compound: 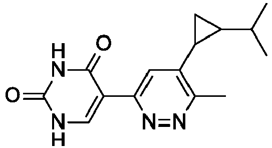 (Formula I, which is 5-[5-[2-isopropylcyclopropyl]-6-methyl-pyridazin-3-yl]-1H-pyrimidine-2,4-dione).

Accordingly, the present invention provides crystalline forms of the compound 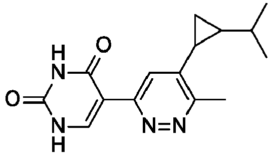 (5-[5-[2-isopropylcyclopropyl]-6-methyl-pyridazin-3-yl]-1H-pyrimidine-2,4-dione).

In a preferred embodiment, the crystalline form is of the compound 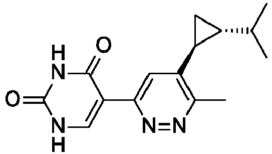 (5-[5-[(1S,2R)-2-isopropylcyclopropyl]-6-methyl-pyridazin-3-yl]-1H-pyrimidine-2,4-dione.

The present invention also provides a pharmaceutical composition comprising a crystalline form of the compound 5-[5-[2-isopropylcyclopropyl]-6-methyl-pyridazin-3-yl]-1H-pyrimidine-2,4-dione, and one or more pharmaceutically acceptable carriers, diluents, or excipients. In a preferred embodiment, the pharmaceutical composition comprises a crystalline form of 5-[5-[(1S,2R)-2-isopropylcyclopropyl]-6-methyl-pyridazin-3-yl]-1H-pyrimidine-2,4-dione, and one or more pharmaceutically acceptable carriers, diluents, or excipients.

### Crystalline Form 1

In one preferred embodiment, the present invention provides a composition comprising at least 500 gm of an anhydrous crystalline form of the compound of the formula: 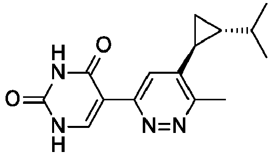 (5-[5-[(1S,2R)-2-isopropylcyclopropyl]-6-methyl-pyridazin-3-yl]-1H-pyrimidine-2,4-dione).

In another preferred embodiment, the anhydrous crystalline form in the composition is characterized by at least one of: (a) a peak in the X-ray diffraction pattern, at a diffraction angle 2-theta of 4.7°, in combination with one or more of the peaks at 11.7°, 15.8 °, 16.0°, 26.8°, and 27.2°; with a tolerance for the diffraction angles of 0.2 degrees; and (b) a ¹³C solid state NMR spectrum which comprises peaks referenced to the highfield resonance of adamantane (δ = 29.5 ppm) at: 25.4, 28.9, 43.1, 109.3, 124.4, 128.2 and 160.7 ppm (± 0.2 ppm, respectively).

In another preferred embodiment, the anhydrous crystalline form is further characterized by a ¹³C solid state NMR spectrum which comprises one or more peaks referenced to the highfield resonance of adamantane (δ = 29.5 ppm) at: 16.6, 18.3, 20.7, 22.1, 23.9, 26.6, 27.6, 28.1, 30.2, 31.2, 32.2, 38.6, 39.7, 41.2, 111.0, 112.0, 130.7, 145.1, 146.3, 150.1, 151.0, 153.4, 156.7, 157.6, 158.6, 159.3, 160.7, 163.4, 165.9, 169.4, and 170.2 ppm (± 0.2 ppm, respectively).

In another preferred embodiment, the anhydrous crystalline form is characterized by a ¹³C solid state NMR spectrum which comprises peaks referenced to the highfield resonance of adamantane (δ = 29.5 ppm) at: 16.6, 18.3, 20.7, 22.1, 23.9, 25.4, 26.6, 27.6, 28.1, 28.9, 30.2, 31.2, 32.2, 38.6, 39.7, 41.2, 43.1, 109.3, 111.0, 112.0, 124.4, 128.2, 130.7, 145.1, 146.3, 150.1, 151.0, 153.4, 156.7, 157.6, 158.6, 159.3, 160.7, 163.4, 165.9, 167.0, 169.4, and 170.2 ppm (± 0.2 ppm, respectively).

In another preferred embodiment, the anhydrous crystalline form in the composition is of the compound 5-[5-[(1S,2R)-2-isopropylcyclopropyl]-6-methyl-pyridazin-3-yl]-1H-pyrimidine-2,4-dione.

In another preferred embodiment, the composition is at least 50%, 60%, 70%, 80%, 90%, 95%, 96%, 97%, 98%, 99% or 100% free of contaminants. In another preferred embodiment, the composition is at least 95% free of contaminants. In another preferred embodiment, the composition is at least 96% free of contaminants. In another preferred embodiment, the composition is at least 97% free of contaminants. In another preferred embodiment, the composition is at least 98% free of contaminants. In another preferred embodiment, the composition is at least 99% free of contaminants.

In another preferred embodiment, the composition is a granulate composition. In another preferred embodiment, the granulate composition is a wet granulate composition. In another preferred embodiment, the granulate composition is a dry granulate composition.

In another preferred embodiment, the present invention provides a manufacturing container comprising at least 1, 2, 3, 4, 5, 6, 7, 8, 9, 10, 20, 30, 40, 50, 60, 70, 80, 90, 100, 200, 300, 400, 500, 600, 700, 800, 900 or 1000 kg of the composition. In another preferred embodiment, the present invention provides a manufacturing container comprising at least 1 kg of the composition. In another preferred embodiment, the present invention provides a manufacturing container comprising at least 5 kg of the composition.

In another preferred embodiment, the present invention provides a pharmaceutical composition comprising the composition, and one or more pharmaceutically acceptable carriers, diluents, or excipients.

### Crystalline Form 2

In another preferred embodiment, the crystalline form is a desolvate (anhydrous) crystalline form of the compound of the formula: 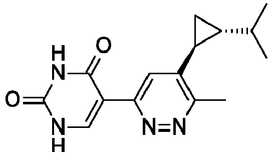 (5-[5-[(1S,2R)-2-isopropylcyclopropyl]-6-methyl-pyridazin-3-yl]-1H-pyrimidine-2,4-dione), which is characterized by at least one of: (a) a peak in the X-ray diffraction pattern, at a diffraction angle 2-theta of 4.3° in combination with one or more of the peaks at 6.4°, 9.6°, 11.0° and 11.6°; with a tolerance for the diffraction angles of 0.2 degrees; and (b) a ¹³C solid state NMR spectrum which comprises peaks referenced to the highfield resonance of adamantane (δ = 29.5 ppm) at: 17.2, 28.7, 122.0, 126.1, 143.6 and 155.5 ppm (± 0.2 ppm, respectively).

In another preferred embodiment, the desolvate (anhydrous) crystalline form is further characterized by a ¹³C solid state NMR spectrum which comprises one or more peaks referenced to the highfield resonance of adamantane (δ = 29.5 ppm) at: 18.9, 21.1, 22.5, 24.4, 26.7, 27.8, 29.7, 39.7, 41.3, 45.5, 50.1, 110.7, 111.7, 112.4, 125.0, 129.1, 144.8, 146.2, 147.2, 149.9, 151.1, 153.0, 155.5, 157.2, 159.0, 160.6, 161.4, 162.7, 164.8, 167.0, 168.3, and 170.4 ppm (± 0.2 ppm, respectively).

In another preferred embodiment, the desolvate (anhydrous) crystalline form is characterized by a ¹³C solid state NMR spectrum which comprises peaks referenced to the highfield resonance of adamantane (δ = 29.5 ppm) at: 17.2, 18.9, 21.1, 22.5, 24.4, 26.7, 27.8, 28.7, 29.7, 39.7, 41.3, 45.5, 50.1, 110.7, 111.7, 112.4, 122.0, 125.0, 126.1, 129.1, 143.6, 144.8, 146.2, 147.2, 149.9, 151.1, 153.0, 155.5, 157.2, 159.0, 160.6, 161.4, 162.7, 164.8, 167.0, 168.3, and 170.4 ppm (+ 0.2 ppm, respectively).

In another preferred embodiment, the desolvate (anhydrous) crystalline form is at least 50%, 60%, 70%, 80%, 90%, 95%, 96%, 97%, 98%, 99% or 100% free of contaminants. In another preferred embodiment, the desolvate (anhydrous) crystalline form is at least 95% free of contaminants. In another preferred embodiment, the desolvate (anhydrous) crystalline form is at least 96% free of contaminants. In another preferred embodiment, the desolvate (anhydrous) crystalline form is at least 97% free of contaminants. In another preferred embodiment, the desolvate (anhydrous) crystalline form is at least 98% free of contaminants. In another preferred embodiment, the desolvate (anhydrous) crystalline form is at least 99% free of contaminants.

In another preferred embodiment, the present invention provides a composition comprising at least 100, 200, 300, 400, 500, or 1000 g of the desolvate (anhydrous) crystalline form. In another preferred embodiment, the present invention provides a composition comprising at least 100 g of the desolvate (anhydrous) crystalline form. In another preferred embodiment, the composition is a granulate composition. In another preferred embodiment, the granulate composition is a wet granulate composition. In another preferred embodiment, the granulate composition is a dry granulate composition.

In another preferred embodiment, the present invention provides a manufacturing container comprising at least 1, 2, 3, 4, 5, 6, 7, 8, 9, 10, 20, 30, 40, 50, 60, 70, 80, 90, 100, 200, 300, 400, 500, 600, 700, 800, 900 or 1000 kg of the desolvate (anhydrous) crystalline form. In another preferred embodiment, the present invention provides a manufacturing container comprising at least 1 kg of the desolvate (anhydrous) crystalline form. In another preferred embodiment, the present invention provides a pharmaceutical composition comprising the desolvate (anhydrous) crystalline form, and one or more pharmaceutically acceptable carriers, diluents, or excipients.

### Crystalline Form 3

In another preferred embodiment, the present application provides a hemihydrate crystalline form of the compound of the formula: 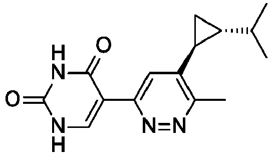 (5-[5-[(1S,2R)-2-isopropylcyclopropyl]-6-methyl-pyridazin-3-yl]-1H-pyrimidine-2,4-dione).

In another preferred embodiment, the hemihydrate crystalline form is characterized by at least one of: (a) a peak in the X-ray diffraction pattern, at a diffraction angle 2-theta of 5.0°, in combination with one or more of the peaks at 16.4°, 17.8 and 26.6°; with a tolerance for the diffraction angles of 0.2 degrees; and (b) a ¹³C solid state NMR spectrum which comprises peaks referenced to the highfield resonance of adamantane (δ = 29.5 ppm) at: 23.5, 34.6, 110.2, 124.1, 126.8 and 166.2 ppm (+ 0.2 ppm, respectively).

In another preferred embodiment, the hemihydrate crystalline form is further characterized by a ¹³C solid state NMR spectrum which comprises one or more peaks referenced to the highfield resonance of adamantane (δ = 29.5 ppm) at: 16.5, 18.9, 19.6, 20.4, 22.6, 24.4, 26.0, 27.4, 28.3, 30.0, 30.9, 38.4, 40.5, 110.8, 130.4, 131.2, 146.0, 147.1, 150.4, 151.2, 153.4, 157.1, 157.7, 158.3, 159.6, 163.1, and 169.7 ppm (± 0.2 ppm, respectively).

In another preferred embodiment, the hemihydrate crystalline form is characterized by a ¹³C solid state NMR spectrum which comprises peaks referenced to the highfield resonance of adamantane (δ = 29.5 ppm) at: 16.5, 18.9, 19.6, 20.4, 22.6, 23.5, 24.4, 26.0, 27.4, 28.3, 30.0, 30.9, 34.6, 38.4, 40.5, 110.2, 110.8, 124.1, 126.8, 130.4, 131.2, 146.0, 147.1, 150.4, 151.2, 153.4, 157.1, 157.7, 158.3, 159.6, 163.1, 166.2, and 169.7 ppm (± 0.2 ppm, respectively).

In another preferred embodiment, the hemihydrate crystalline form is at least 50%, 60%, 70%, 80%, 90%, 95%, 96%, 97%, 98%, 99% or 100% free of contaminants. In another preferred embodiment, the hemihydrate crystalline form is at least 95% free of contaminants. In another preferred embodiment, the hemihydrate crystalline form is at least 96% free of contaminants. In another preferred embodiment, the hemihydrate crystalline form is at least 97% free of contaminants. In another preferred embodiment, the hemihydrate crystalline form is at least 98% free of contaminants. In another preferred embodiment, the hemihydrate crystalline form is at least 99% free of contaminants.

In another preferred embodiment, the present invention provides a composition comprising at least 100, 200, 300, 400, 500, or 1000 g of the hemihydrate crystalline form. In another preferred embodiment, the present invention provides a composition comprising at least 100 g of the hemihydrate crystalline form.

In another preferred embodiment, the composition is a granulate composition. In another preferred embodiment, the granulate composition is a wet granulate composition. In another preferred embodiment, the granulate composition is a dry granulate composition.

In another preferred embodiment, the present invention provides a manufacturing container comprising at least 1, 2, 3, 4, 5, 6, 7, 8, 9, 10, 20, 30, 40, 50, 60, 70, 80, 90, 100, 200, 300, 400, 500, 600, 700, 800, 900 or 1000 kg of the hemihydrate crystalline form. In another preferred embodiment, the present invention provides a manufacturing container comprising at least 1 kg of the hemihydrate crystalline form.

In another preferred embodiment, the present invention provides a pharmaceutical composition comprising the hemihydrate crystalline form, and one or more pharmaceutically acceptable carriers, diluents, or excipients.

### Methods of Treatment

The present invention also provides the crystalline compound for use in a method for treating cancer, comprising administering to a patient in need thereof an effective amount of a crystalline form of the compound 5-[5-[2-isopropylcyclopropyl]-6-methyl-pyridazin-3-yl]-1H-pyrimidine-2,4-dione.

In another preferred embodiment, the cancer is bladder cancer, breast cancer, cholangiocarcinoma, colorectal cancer, colon cancer, gastric cancer, gallbladder cancer, glioblastoma, head and neck cancer, liver cancer, lung cancer, lymphoma, medulloblastoma, melanoma, ovarian cancer, pancreatic cancer, prostate cancer or renal cancer. In one embodiment, the breast cancer is triple-negative breast cancer. In another embodiment, the lung cancer is non-small cell lung cancer.

The present invention also provides a crystalline form of the compound 5-[5-[2-isopropylcyclopropyl]-6-methyl-pyridazin-3-yl]-1H-pyrimidine-2,4-dione, for use in therapy.

The present invention also provides a crystalline form of the compound 5-[5-[2-isopropylcyclopropyl]-6-methyl-pyridazin-3-yl]-1H-pyrimidine-2,4-dione, for use in the treatment of cancer. In a preferred embodiment, the crystalline form is of the compound 5-[5-[(1S,2S)-2-ethylcyclopropyl]-6-methyl-pyridazin-3-yl]-1H-pyrimidine-2,4-dione.

The present invention also provides a crystalline form of the compound 5-[5-[2-isopropylcyclopropyl]-6-methyl-pyridazin-3-yl]-1H-pyrimidine-2,4-dione, in the manufacture of a medicament for the treatment of cancer.

Subjects that can benefit from CD73 inhibitor treatment include those with tumors resistant or refractory to the anti-PD1/PDL-1 inhibitors, such as non-small cell lung cancer, bladder cancer, and melanoma; those with EGFR/BRAF/Kras mutant cancers, such as non-small cell lung cancer, bladder cancer, melanoma, colon, and pancreas; those with estrogen receptor (-) cancers, such as triple negative breast cancer; subjects with high expression level of CD73, such as pancreatic cancer and colorectal cancer. If desired, such subject can be selected for therapy with a compound disclosed herein, or a pharmaceutically acceptable salt thereof, based on the presence of high CD73 expression levels in their tumors, as measured by an IHC assay; or based on the presence of EGFR and BRAF mutations in their tumors as detected by RT-PCR assays; or based on the loss of estrogen receptor in their tumors, as detected by an IHC or RT-PCR assay; or based on the presence of high levels of adenosine and AMP in their tumors or plasma as detected by an LC-MS assay. For pharmacodynamic assessment, an LC-MS based ex vivo assay described herein can be used to measure the effect of a CD73 inhibitor on the conversion of AMP to adenosine in the blood.

In a preferred embodiment, the patient is one in whom the serum CD73 activity has been determined. In one preferred embodiment, "determining CD73 activity" means determining if CD73 activity is present. Methods for determining the level of CD73 expression or CD73 activity are known to those of ordinary skill in the art, *e.g.,* see S Morello, et al., J. Trans. Med. 2017; 15:244. In another preferred embodiment, "determining CD73 activity" means quantifying the level of AMP conversion to adenosine by CD73, and an LC-MS based assay is provided herein that facilitates quantifying the level of CD73 activity.

In another preferred embodiment, the patient is one in whom CD73 expression in tissue has been determined. In another embodiment, the tissue is tumor tissue. Methods for determining the level of CD73 expression in tissue are known to those of ordinary skill in the art, *e.g.,* using western blotting or immunohistochemistry (X-R Wu, et al., J. Surg. Oncol. 2012; 106: 130-137).

In one embodiment, the present invention provides the crystalline compound for use in a method of treating cancer, comprising administering an effective amount of crystalline 5-[5-[2-isopropylcyclopropyl]-6-methyl-pyridazin-3-yl]-1H-pyrimidine 2,4-dione in simultaneous, separate, or sequential combination with one or more anti-tumor agents. Non-limiting examples of anti-tumor agents include ramucirumab, necitumumab, olaratumab, gemcitabine, pemetrexed, galunisertib, abemaciclib, gefitinib, vemurafenib, dabrafenib, trametinib, cisplatin, carboplatin, dacarbazine, liposomal doxorubicin, docetaxel, cyclophosphamide and doxorubicin, navelbine, eribulin, paclitaxel, paclitaxel protein-bound particles for injectable suspension, ixabepilone, capecitabine, FOLFOX (leucovorin, fluorouracil, and oxaliplatin), FOLFIRI (leucovorin, fluorouracil, and irinotecan), cetuximab, an EGFR inhibitor, a Raf inhibitor, a B-Raf inhibitor, an ERK inhibitor, a CDK4/6 inhibitor, an idoleamine 2,3-dioxygenase inhibitor, a TGFβ inhibitor, and a TGFβ receptor inhibitor.

In a another embodiment, the present invention provides the crystalline compound for use in a method of treating cancer, comprising administering an effective amount of crystalline 5-[5-[2-isopropylcyclopropyl]-6-methyl-pyridazin-3-yl]-1H-pyrimidine 2,4-dione in simultaneous, separate, or sequential combination with one or more immuno-oncology agents. In one preferred embodiment, the immune-oncology agent is an anti-PD-1 antibody, an anti-PD-L1 antibody, an anti-CD137 agonist antibody, or an anti-CTLA4 antibody. Non-limiting examples of immuno-oncology agents include nivolumab, ipilimumab, pidilizumab, pembrolizumab, tremelimumab, urelumab, lirilumab, atezolizumab, durvalumab, and the anti-PD-L1 antibody LY3300054 (the heavy and light chain sequences of which are forth in WO 2017/034916 and US 2017/0058033 as SEQ ID NOS: 10 and 11, respectively). In one preferred embodiment, the immune-oncology agent is an anti-PD-1 antibody. In another preferred embodiment, the immune-oncology agent is an anti-PD-L1 antibody. In another preferred the immune-oncology agent is LY3300054.

In one embodiment, the present invention provides the crystalline compound for use in a method of treating non-small cell lung cancer, comprising administering an effective amount of crystalline 5-[5-[2-isopropylcyclopropyl]-6-methyl-pyridazin-3-yl]-1H-pyrimidine 2,4-dione in simultaneous, separate, or sequential combination with another agent. In one preferred embodiment, the other agent is osimertinib, cetuximab or abemaciclib. In another preferred embodiment, the other agent is osimertinib. In another preferred embodiment, the other agent is cetuximab. In another preferred embodiment, the other agent is abemaciclib.

In another preferred embodiment, the present invention provides the crystalline compound for use in a method of treating melanoma, comprising administering an effective amount of crystalline 5-[5-[2-isopropylcyclopropyl]-6-methyl-pyridazin-3-yl]-1H-pyrimidine 2,4-dione in simultaneous, separate, or sequential combination with another agent. In one preferred embodiment, the other agent is a BRAF inhibitor, an anti-PD-1 antibody, or an anti-PD-L1 antibody. In another preferred embodiment, the other agent is a BRAF inhibitor. In another preferred embodiment, the other agent is an anti-PD-1 antibody. In another preferred embodiment, the other agent is an anti-PD-L1 antibody. In another preferred embodiment, the anti-PD-L1 antibody is LY3300054 (the heavy and light chain sequences of which are forth in WO 2017/034916 and US 2017/0058033 as SEQ ID NOS: 10 and 11, respectively).

In another preferred embodiment, the present invention provides the crystalline compound for use in a method of treating colorectal cancer, comprising administering an effective amount of crystalline 5-[5-[2-isopropylcyclopropyl]-6-methyl-pyridazin-3-yl]-1H-pyrimidine 2,4-dione in simultaneous, separate, or sequential combination with another agent. In one preferred embodiment, the other agent is abemaciclib.

In another preferred embodiment, the present invention provides a crystalline compound for use in a method of treating pancreatic cancer, comprising administering an effective amount of crystalline 5-[5-[2-isopropylcyclopropyl]-6-methyl-pyridazin-3-yl]-1H-pyrimidine 2,4-dione in simultaneous, separate, or sequential combination with another agent. In one preferred embodiment, the other agent is abemaciclib.

In another preferred embodiment, the present invention provides a crystalline compound for use in a method of treating triple-negative breast cancer, comprising administering an effective amount of crystalline 5-[5-[2-isopropylcyclopropyl]-6-methyl-pyridazin-3-yl]-1H-pyrimidine 2,4-dione in simultaneous, separate, or sequential combination with another agent.

As used above, and throughout the description of the invention, the following terms, unless otherwise indicated, shall be understood to have the following meanings:
A "pharmaceutically acceptable carrier, diluent, or excipient" is a medium generally accepted in the art for the delivery of biologically active agents to mammals, e.g., humans.

The terms "treatment," "treat," "treating," and the like, are meant to include slowing or reversing the progression of a disorder. These terms also include alleviating, ameliorating, attenuating, eliminating, or reducing one or more symptoms of a disorder or condition, even if the disorder or condition is not actually eliminated and even if progression of the disorder or condition is not itself slowed or reversed.

"Effective amount" means the amount of the compound, or pharmaceutically acceptable salt thereof, of the present invention or pharmaceutical composition containing a compound, or pharmaceutically acceptable salt thereof, of the present invention that will elicit the biological or medical response of or desired therapeutic effect on a patient by a treating clinician. In one embodiment, the compound, or a pharmaceutically acceptable salt thereof, inhibits the conversion of AMP to adenosine in an *in vitro* or *ex vivo* CD73 enzyme assay. In another embodiment, the compound, or a pharmaceutically acceptable salt thereof, inhibits the conversion of AMP to adenosine in mouse whole blood from animals treated with different doses of the compound.

As used herein, the term "patient" refers to a human.

An effective amount can be readily determined by the attending diagnostician, as one skilled in the art, by the use of known techniques and by observing results obtained under analogous circumstances. In determining the effective amount for a patient, a number of factors are considered by the attending diagnostician, including, but not limited to: the species of patient; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

The compounds of the present invention are preferably formulated as pharmaceutical compositions administered by any route which makes the compound bioavailable, including oral, intravenous, and transdermal routes. Most preferably, such compositions are for oral administration. Such pharmaceutical compositions and processes for preparing same are well known in the art. (See, e.g., Remington: The Science and Practice of Pharmacy (D.B. Troy, Editor, 21st Edition, Lippincott, Williams & Wilkins, 2006).

It will be understood by the skilled artisan that compounds of the present invention are capable of forming salts. The compounds of the present invention contain basic heterocycles, and accordingly react with any of a number of inorganic and organic acids to form pharmaceutically acceptable acid addition salts. Such pharmaceutically acceptable acid addition salts and common methodology for preparing them are well known in the art. *See, e.g.,* P. Stahl, et al., HANDBOOK OF PHARMACEUTICAL SALTS: PROPERTIES, SELECTION AND USE, (VCHA/Wiley-VCH, 2008)
As used herein, "pharmaceutically acceptable salts" or "a pharmaceutically acceptable salt" refers to the relatively non-toxic, inorganic and organic salt or salts of the compound of the present invention (S.M. Berge, et al., "Pharmaceutical Salts", Journal of Pharmaceutical Sciences, Vol 66, No. 1, January 1977).

As used herein, "granulate composition" refers to a composition in granular form which, in the pharmaceutical manufacturing process, is a predecessor composition to a pharmaceutical composition.

As used herein, "manufacturing container" refers to a container that is employed in the manufacture of a pharmaceutical container, but not in the medicinal chemistry laboratory. Examples of manufacturing containers include, but are not limited to, a hopper collector, a bed, a dryer bed, a granulator bed, a dryer tray, a granulator bucket, and a mixing bowl.

Compounds of Formula I can be prepared according to synthetic methods well known and appreciated in the art. Suitable reaction conditions for the steps of these reactions are well known in the art and appropriate substitutions of solvents and co-reagents are within the skill of the art. Likewise, it will be appreciated by those skilled in the art that synthetic intermediates may be isolated and/or purified by various well known techniques as needed or desired, and that frequently, it will be possible to use various intermediates directly in subsequent synthetic steps with little or no purification. Furthermore, the skilled artisan will appreciate that in some circumstances, the order in which moieties are introduced is not critical. The particular order of steps required to produce the compounds of the present invention is dependent upon the particular compound being synthesized, the starting compound, and the relative liability of the substituted moieties, as is well appreciated by the skilled chemist. All substituents, unless otherwise indicated, are as previously defined, and all reagents are well known and appreciated in the art.

As used herein, the following terms have the meanings indicated: "ACN" refers to acetonitrile; "DAST" refers to diethylaminosulfur trifluoride, "DCM" refers to dichloromethane; "DMAP" refers to 4-dimethylaminopyridine ; "dmso" or "DMSO" refers to dimethyl sulfoxide; "ee" refers to enantiomeric excess; "ES/MS" refers to Electrospray Mass Spectrometry; "EtOAc" refers to ethyl acetate; "Et₂O" refers to diethyl ether; "FBS" refers to fetal bovine serum; "GC-MS" refers to Gas Chromatography-Mass Spectometry; "HBSS" refers to Hank's Balanced Salt Solution; "IC₅₀" refers to half maximal inhibitory concentration; "LAH" refers to lithium aluminum hydride; "LC-ES/MS" refers to Liquid Chromatography Electrospray Mass Spectometry; "MS" refers to Mass Spectometry; "MeOH" refers to methanol; "MTBE" refers to methyl tert-butyl ether; "nBuLi" refers to *n*-butyllithium; "nm" refers to nanometer or nanometers; "NMR" refers to nuclear magnetic resonance; "OAc" refers to acetate; "psi" refers to pounds per square inch; "RT" refers to room temperature or ambient temperature; "SCX" refers to StrongCation Exchange; "SFC" refers to Super-critical Fluid Chromatography; "S_{N}Ar" refers to nucleophilic aromatic substitution; " TEA" refers to triethylamine; "THF" refers to tetrahydrofuran; "t_{R}" refers to retention time; and "w/w" refers to weight/weight proportions in solution.

Compounds of Formula I may be synthesized as illustrated in the following schemes.

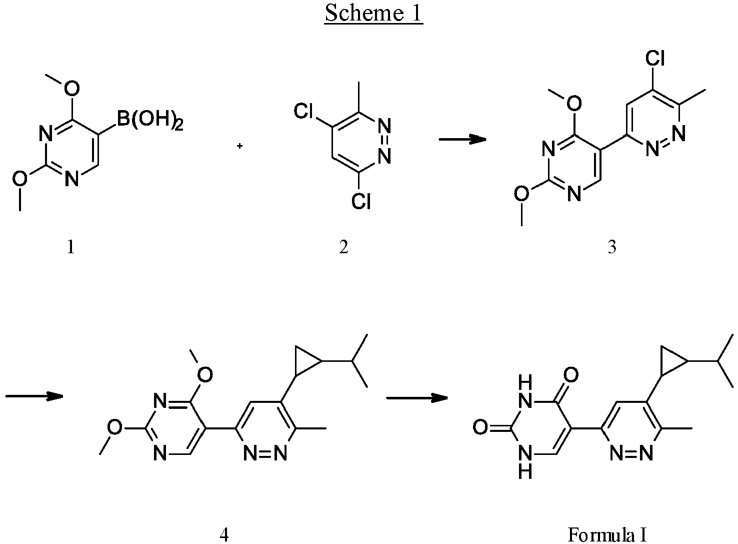

Scheme 1 depicts the preparation of the compounds of Formula I. Using well-known Suzuki-type conditions, commercially available (2,4-dimethoxypyrimidin-5-yl)boronic acid 1 may be coupled to 4,6-dichloro-3-methyl-pyridazine. Subsequent Suzuki-coupling of the chloro moiety of 3 with an appropriately substituted trans-cyclopropylboronate may be accomplished to obtain 4. The skilled artisan will recognize that the enantiomers of compound 4 may be separated using chiral separation techniques well known in the art. Compounds of Formula I may be prepared by deprotection of the methoxy groups in 4 under an array of demethylation conditions well described in the art.

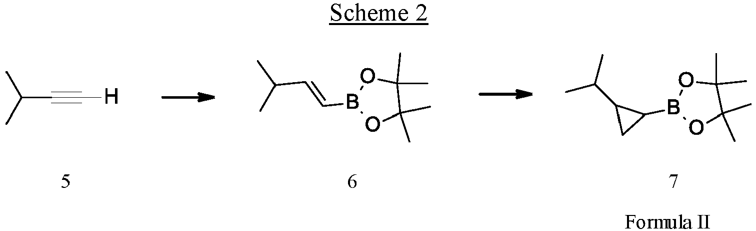

Scheme 2 depicts the requisite cyclopropyl boronic esters needed to prepare compounds of Formula II. The skilled artisan will recognize that hydroboration of the appropriately substituted alkyne 5 may be effected under an array of conditions, especially in the presence of a transition-metal catalyst such as Cu or Zr, to obtain the alkenyl boronate 6. The alkenyl boronate may be cyclopropanated under stabilized carbenoid-type conditions well known in the art, such as Simmons-Smith cyclopropanation, Corey-Chaykovsky reaction, and the method of cyclopropanation with diazomethane (or diazo compounds) both with (*e.g.,* Cu, Pd, or Ni) and without (*e.g.,* thermally or photochemically) transition metal catalysts, to obtain the appropriately substituted trans-cyclopropylboronate 7. The skilled artisan will recognize that the thermodynamically favored cyclopropanation product will be a mixture of trans enantiomers in 7.

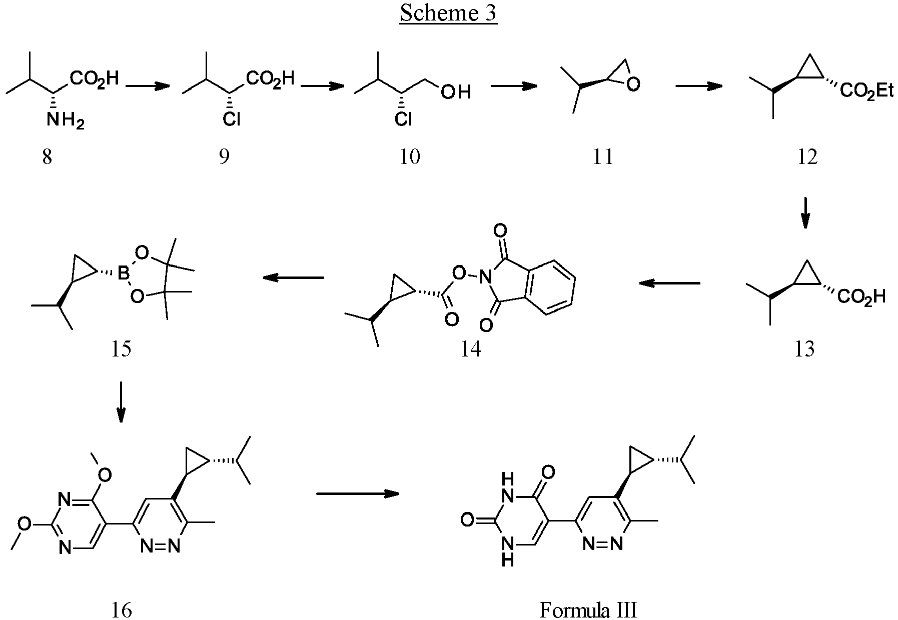

Scheme 3 depicts the chiral synthesis of compounds of Formula III. Diazotization of the appropriately substituted amino acid 8 under modified Sandmeyer conditions (radical S_{N}Ar) to give 9 is well known in the art. Subsequent reduction to alcohol 10 may be accomplished utilizing an array of reducing agents well known in the art, including aluminum hydrides and diborane as reducing agents. Cyclization to the chiral epoxide 11 under basic conditions is well described in the art. A stereoselective Homer-Wadsworth-Emmons reaction may be used by treating the chiral epoxide 11 with a suitable phosphonate ester (e.g., ethyl 2-diethoxyphosphorylacetate or triethylphosphonoacetate) and suitable base (e.g., alkyllithium, metal alkoxides, or metal hydrides) to obtain the trans-cyclopropane derivative 12 (See, *e.g.,* L. Delhaye; A. Merschaert; P. Delbeke;W. Brione. Org. Proc. Res. & Dev. 2007, 11, 689-692.) Hydrolysis of compound 12 to the corresponding acid 13 may be accomplished under a wide array of basic conditions well described in the art. Subsequent coupling of acid 13 with an appropriately substituted N-hydroxy phthalimide is well described in the art, utilizing a suitable acid-activating agent, e.g., carbonyldiimidazole, in the presence of a mild non-nucleophilic base, to prepare compound 14. Decarboxylative borylation of the N-hydroxypthalimide ester 14 may be accomplished under many conditions known in the art, including in the presence of a transition metal catalyst (*e.g*., Suzuki-Miyaura reaction), under photolytic conditions, or by single electron transfer reactions, including, for example, complexation of the N-hydroxyphthalimide ester with diboron in a radical coupling facilitated by a pyridine-boron radical, to obtain the trans-cyclopropane boronate 15. (See, *e.g*., W.-M. Cheng; S. Rui; B. Zhao; W.-L. Xing; Y. Fu. Org. Lett. 2017, 19, 4291-4294.) Coupling of the boronate 15 with arylchloride 4 and subsequent demethylation may be performed similarly to that described in Scheme 1 to obtain the chiral compound types of Formula III.

### Preparations and Examples

The following Preparations and Examples further illustrate the invention and represent typical synthesis of the compounds of the invention, but should not be construed to limit the scope of the invention in any way. The reagents and starting materials are readily available or may be readily synthesized by one of ordinary skill in the art. It should be understood that the Preparations and Examples are set forth by way of illustration and not limitation, and that various modifications may be made by one of ordinary skill in the art.

LC-ES/MS is performed on an Agilent HP1100 liquid chromatography system. Electrospray mass spectrometry measurements (acquired in positive and/or negative mode) are performed on a Mass Selective Detector quadrupole mass spectrometer interfaced to the HP1100 HPLC. LC-MS conditions (low pH): column: PHENOMENEX^{®} GEMINI^{®} NX C-18 2.1 × 50 mm 3.0 µm; gradient: 5-100% B in 3 min, then 100% B for 0.75 min column temperature: 50 °C +/-10 °C; flow rate: 1.2 mL/min; Solvent A: deionized water with 0.1% HCOOH; Solvent B: ACN with 0.1% formic acid; wavelength 214 nm. Alternate LC-MS conditions (high pH): column: WATERS^{™} XTERRA^{®} MS C-18 columns 2.1 × 50 mm, 3.5 µm; gradient: 5% of solvent A for 0.25 min, gradient from 5% to 100% of solvent B in 3 min and 100% of solvent B for 0.5 min or 10% to 100% of solvent B in 3 min and at 100% of solvent B for 0.75 min; column temperature: 50 °C +/-10 °C; flow rate: 1.2 mL/min; Solvent A: 10 mM NH₄HCO₃ pH 9; Solvent B: ACN ; wavelength: 214 nm.

Preparative reversed phase chromatography is performed on an Agilent 1200 LC-ES/MS equipped with a Mass Selective Detector mass spectrometer and a Leap autosampler/fraction collector. High pH methods are run on a 75 × 30 mm PHENOMENEX^{®} GEMINI^{®}-NX, 5 µm particle size column with a 10 × 20 mm guard. Flow rate of 85 mL/min. Eluent is 10 mM ammonium bicarbonate (pH 10) in ACN.

NMR spectra are performed on a Bruker A VIII HD 400 MHz NMR Spectrometer, obtained as CDCl₃ or (CD₃)₂SO solutions reported in ppm, using residual solvent [CDCl₃, 7.26 ppm; (CD₃)₂SO, 2.50 ppm] as a reference standard. When peak multiplicities are reported, the following abbreviations may be used: s (singlet), d (doublet), t (triplet), q (quartet), m (multiplet), br-s (broad singlet), dd (doublet of doublets), dt (doublet of triplets). Coupling constants (J), when reported, are reported in hertz (Hz).

### Preparation 1

### 4,4, 5, 5-Tetramethyl-2-[(E)-3-methylbut-1-enyl]-1,3,2-dioxaborolane

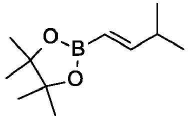

Add pinacol borane (9.5 ml, 64 mmol) drop wise over 5 minutes to ice cold 3-methylbut-1-yne (4.91 g, 72.0 mmol). Seal pressure vessel, warm to RT and stir 18 hours. Add bis(cyclopentadienyl)zirconium(IV) chloride hydride (2.0 g, 7.4 mmol) and TEA (1.1 mL, 7.9 mmol). Seal the pressure vessel, place in a 60 °C oil bath, and stir for 10 minutes. Cool the resulting red solution to RT for 2.5 hours. Dilute the reaction mixture with DCM (200 mL), wash with saturated aqueous NaHCO₃ (100 mL), saturated aqueous NaCl (50 mL), dry over MgSO₄, filter through a pad of silica gel (150 mL), rinse silica gel with DCM (700 mL), and concentrate *in vacuo* to afford the title compound (11.5 g, 82%). ES/MS (m/z): 196 (M+H). ¹H NMR (CDCl₃) δ: 1.03 (d, J= 6.7 Hz, 6H), 1.29 (s, 12H), 2.37 (m, 1H), 5.40 (dd, 1H), 6.64 (dd, 1H).

### Preparation 2

### Rac-trans-2-[2-isopropylcyclopropyl]-4,4,5,5-tetramethyl-1,3,2-dioxaborolane

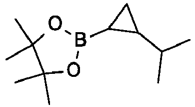

Portion wise, add N-nitroso-N-methylurea to an ice cold biphasic mixture of Et₂O (70 mL) and aqueous KOH (30.5 g, 435 mmol, 70 mL H₂O). Stir until solids dissolve (< 5 minutes). Pipet the resulting diazomethane solution into a rapidly stirring ice cold suspension of Pd(OAc)₂ (237 mg, 1.05 mmol) and 4,4,5,5-tetramethyl-2-[(E)-3-methylbut-1-enyl]-1,3,2-dioxaborolane (4.00 g, 20.4 mmol) in Et₂O (70 mL). After complete addition, warm the reaction mixture to RT, filter through diatomaceous earth, and concentrate the filtrate *in vacuo.* Dissolve the resulting residue in DCM, filter through a plug of silica gel (25 g), and concentrate *in vacuo* to obtain the title compound (4.32 g, >99%). ES/MS (m/z): 210 (M+H). ¹H NMR (CDCl₃) δ: -0.35 (m, 1H), 0.45 (m, 1H), 0.65 (m, 1H), 0.78 (m, 1H), 0.98 (m, 7H), 1.23 (s, 12H).

### Preparation 3

### 4-Chloro-6-(2,4-dimethoxypyrimidin-5-yl)-3-methyl-pyridazine

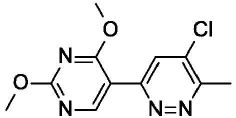

In a pressure vial, combine 2,4-dimethoxy-5-pyrimidinylboronic acid (6.75 g, 36.7 mmol), 4,6-dichloro-3-methyl-pyridazine (5.98 g, 36.7 mmol), [1,1'-bis (diphenylphosphino)ferrocene]dichloropalladium (II) (0.55 g, 0.73 mmol), Cs₂CO₃ (29.9 g, 91.8 mmol) in a 4:1 mixture of 1,4-dioxane/H₂O (151 mL). Evacuate the air and backfill with N₂. Seal the vessel and heat at 70 °C for 3 hours. Filter the residue over diatomaceous earth and rinse with EtOAc. Wash the organic mixture with water followed by saturated aqueous NaCl, dry over MgSO₄, and evaporate to dryness. Purify the resulting black residue by chromatography over silica gel, using a 330 g REDISEP^{®} column with a gradient of 0-30% DCM/(33% MeOH in DCM) over 15 minutes at a flow rate of 200 mL/minute, to afford the title compound (6.2 g, 63%) after evaporation of the chromatographic fractions. ES/MS (m/z) (³⁵Cl/³⁷Cl) 267/269 [M+1]⁺ H NMR (d₆-DMSO) δ: 2.74 (s, 3H), 4.00 (s, 3H), 4.03 (s, 3H), 8.20 (s, 1H), 8.87 (s, 1H).

### Alternate Procedure for Preparation 3

Pass a stream of nitrogen through a mixture of (2,4-dimethoxypyrimidin-5-yl)boronic acid (85 g, 439 mmol), 4,6-dichloro-3-methyl-pyridazine (75 g, 437 mmol) and Cs₂CO₃ (358 g, 1099 mmol) in 1,4-dioxane (1175 mL) and H₂O (340 mL) for 5 minutes. Add [1,1'-bis(diphenylphosphino)ferrocene]dichloropalladium(II) (6.6 g, 8.7 mmol,) and stir the resulting mixture at 75 °C for 16 hours. Cool the reaction to RT, filter the mixture through diatomaceous earth, and rinse the filter cake with EtOAc. Separate the resulting layers and wash the organic phase twice with saturated aqueous NaCl, dry over MgSO₄, filter, and concentrate *in vacuo.* Add water (500 mL) to the resulting residue, stir for 16 hours at RT, and filter the resulting solid. Wash collected solids with H₂O and dry under vacuum for 16 hours to obtain the desired compound (70 g, 54%) as a brown solid. ES/MS (m/z) (³⁵Cl/³⁷Cl) 267/269 [M+1]⁺

### Preparation 4

### trans-5-[2-Isopropylcyclopropyl]-6-methyl-pyridazin-3-yl]-2,4-dimethoxy-pyrimidine

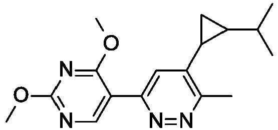

Combine 4-chloro-6-(2,4-dimethoxypyrimidin-5-yl)-3-methyl-pyridazine (1.97 g, 7.39 mmol), rac-trans- 2-[2-isopropylcyclopropyl]-4,4,5,5-tetramethyl-1,3,2-dioxaborolane (3.32 g, 15.8 mmol), bis(di-tert-butyl(4-dimethylaminophenyl)phosphine) dichloropalladium (II) (1.35 g, 1.85 mmol), 1,4-dioxane (37 mL), and 1M aqueous Na₂CO₃ (18 mL, 18 mmol). Purge reaction vessel with N₂ and heat to 90 °C for 18 hours. Cool to RT, dilute with EtOAc (150 mL), and separate layers. Wash the organic layer sequentially with 1M aqueous Na₂CO₃, saturated aqueous NaCl, dry over MgSO₄, filter, and concentrate the filtrate *in vacuo.* Purify the resulting residue by chromatography on silica, eluting with a gradient of 60-100% EtOAc/DCM, to afford the title compound as an essentially racemic mixture of isomers (1.48 g, 64%), after evaporation of the chromatographic fractions.

The isomers are subjected to purification by SFC (Column: PHENOMENEX^{®} LUX^{®} Cellulose-4, 4.6 × 150 mm; 40% MeOH/CO₂ isocratic; flow rate: 5 mL/minutes, UV 250 nm) to afford 647 mg isomer 1: t_{R} = 2.56 min and 647 mg isomer 2: t_{R} = 3.75 min. ES/MS (m/z): 315 (M+H).

### Preparation 5

### (2S)-2-Chloro-3-methyl-butanoic acid

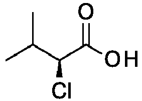

Cool a solution of L-valine (286 g, 2.44 mol) and a 5M aqueous solution of HCl (3.25 L, 16.3 mol) at 0 °C. Add dropwise a 4M aqueous solution of NaNO₂ (1 L, 4 mol) over 2 hours, keeping the internal temperature below 5 °C. Stir the reaction for 2 hours while warming to RT, and stir an additional 16 hours at RT. Portion wise over 30 minutes, add Na₂CO₃ (242 g, 2.28 mol). Extract the resulting solution with MTBE (3 × 1000 mL), wash the combined organic extracts with saturated aqueous NaCl (500 mL), dry the organic extracts over MgSO₄, and concentrate *in vacuo.* Purify the resulting residue by vacuum distillation (15 mbar/140 °C) to afford the title compound (248 g, 68%) as oil. ¹H NMR (400 MHz, CDCl₃) δ: 1.1 (dt, J=6.6, 2.6 Hz, 6H), 2.34-2.42 (m, 1H), 4.19-4.23 (m, 1H), 10.0-12.0 (br-s, 1H).

### Preparation 6

### (2S)-2-Chloro-3-methyl-butan-1-ol

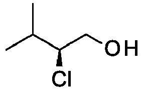

Cool a solution of (2S)-2-chloro-3-methyl-butanoic acid (137 g, 1 mol) in 2-methyltetrahydrofuran (500 mL) to 0 °C. Add a 2.3M solution of LAH in methyltetrahydrofuran (480 mL, 1.1 mol) drop wise over 2.5 hours, keeping the internal temperature below 10 °C. Warm to RT and stir the mixture for 1 hour at RT and 1 hour at 50 °C. Cool the reaction to 0 °C and sequentially and slowly add H₂O (1.48 mL), 15% aqueous solution of NaOH (1.48 mL) and H₂O (4.46 mL). Allow the mixture to warm to RT, filter through a bed of diatomaceous earth, and evaporate the solvent *in vacuo* to afford title compound (110 g, 81%) as colorless oil. ¹H NMR (400 MHz, CDCl₃) δ: 0.97-1.1 (m, 6H), 1.94-2.18 (m, 1H), 2.60-3.09 (br-s, 1H), 3.71-3.78 (m, 1H), 3.80-3.85 (m, 1H), 3.90-3.96 (m, 1H).

### Preparation 7

### (2R)-2-Isopropyloxirane

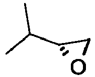

Cool a solution of KOH (195 g, 3.48 mol) in H₂O (195 mL) to 0 °C and add neat (2S)-2-chloro-3-methyl-butan-1-ol (110 g, 801 mmol) over 20 minutes while keeping the internal temperature below 5 °C. Allow the reaction mixture to warm to RT. Purify the reaction mixture by vacuum distillation at 100 mbar, warming from 23 °C to 50 °C, to afford title compound (47 g, 64%) as colorless oil. ¹H NMR (400 MHz, CDCl₃) δ: 0.98 (d, J=6.9 Hz, 3H), 1.05 (d, J=6.9 Hz, 3H), 1.51 (o, J=6.9 Hz, 1H), 2.52-2.54 (m, 1H), 2.70-2.75 (m, 2H).

### Preparation 8

### Ethyl (1S,2R)-2-isopropylcyclopropanecarboxylate

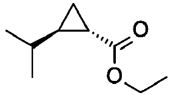

Add a 2.5M solution of nBuLi in hexanes (310 mL, 780 mmol) to a solution of ethyl 2-diethoxyphosphorylacetate (153 mL, 772 mmol) in 1,4-dioxane (870 mL) cooled in an ice/water bath (inner temperature: 8 °C) dropwise over 25 minutes. Warm to RT and stir for 40 minutes. Transfer the solution via cannula to a 3 L pressure vessel and add (2R)-2-isopropyloxirane (70 g, 772 mmol) in 1,4-dioxane (180 mL). Stir the reaction mixture at 150 °C at 50 psi pressure for 14 hours. Cool the reaction mixture to RT and add H₂O (700 mL). Separate the layers and re-extract aqueous phase with MTBE (2 × 500 mL). Combine the organic phases, wash with saturated aqueous NaCl (2 × 350 mL), dry over MgSO₄, and concentrate *in vacuo* to obtain the crude title compound (107.7 g, >99%) as yellow oil, suitable for subsequent use without further purification. GC-MS (m/z): 156 (M+).

### Preparation 9

### (1S,2R)-2-Isopropylcyclopropanecarboxylic acid

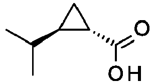

Stir a mixture of ethyl (1S,2R)-2-isopropylcyclopropanecarboxylate (107.7 g, 482.6 mmol) in 1,4-dioxane (800 mL) containing a 25% aqueous solution of NaOH (800 mL) at 100 °C for 7 hours. Cool the mixture to RT, add water (300 mL), extract with MTBE (2 × 500 mL), and discard the organic phase. Acidify the aqueous phase with a 37% aqueous solution of HCl (approximately 500 mL) until pH ~ 1-2.

Extract the acidified aqueous mixture with MTBE (2 × 600 mL), wash the organic layer with saturated aqueous NaCl, dry over MgSO₄, and concentrate *in vacuo,* to yield the title compound (54.2 g, 75%) as amber oil. ¹H NMR (400 MHz, CDCl₃) δ: 0.81-0.86 (m, 1H), 1.01 (dd, J=5.9, 3.7 Hz, 6H), 1.04-1.13 (m, 1H), 1.20-1.24 (m, 1H), 1.28-1.37 (m, 1H), 1.39-1.44 (m, 1H).

### Preparation 10

### (1,3-Dioxoisoindolin-2-yl) (1S,2R)-2-isopropylcyclopropanecarboxylate

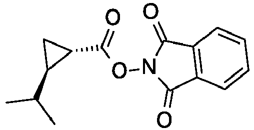

Stir a suspension of (1S,2R)-2-isopropylcyclopropanecarboxylic acid (54.2 g, 359 mmol), 2-hydroxyisoindoline-1,3-dione (59.8 g, 359 mmol) and DMAP (4.44 g, 35.9 mmol) in DCM (690 mL) at 0 °C. Add N,N'-diisopropylcarbodiimide (50.4 g, 395 mmol) drop wise, warm to RT, and stir the resulting reaction mixture for 2 hours at RT. Add H₂O (600 mL) and separate the phases. Extract the aqueous phase with DCM (2 × 300 mL), combine the organic phases, dry over MgSO₄, filter, and evaporate. Purify the resulting solid residue by chromatography on silica, eluting with 100% DCM, to afford
the title compound (96 g, 88%) as pale yellow solid, after evaporation of the chromatographic fractions. ES/MS (m/z): 274 (M+1).

### Preparation 11

### 2-[(1S,2S)-2-Isopropylcyclopropyl]-4,4,5,5-tetramethyl-1,3,2-dioxaborolane

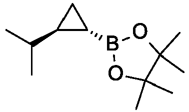

Pass a stream of nitrogen through a solution of (1,3-dioxoisoindolin-2-yl) (1S,2S)-2-isopropylcyclopropanecarboxylate (96 g, 316 mmol) and bis(pinacolato)diboron (160 g, 630 mmol) in EtOAc (480 mL) for 15 minutes. Stir the mixture at 85 °C and add ethyl isonicotinate (24.38 g, 157 mmol) drop wise over 10 minutes. Stir the resulting mixture at 85 °C for 16 hours. Cool the resulting suspension, filter and discard the solids, and evaporate the brown filtrate under reduced pressure. Filter the crude residue over a silica gel plug, eluting with 2% EtOAc/hexanes. Remove the solvent from the filtrate and repurify the resulting residue using chromatography over silica gel, eluting with 3% EtOAc/hexanes, to obtain the title compound (25.3 g, 38%) as colorless oil, after solvent removal from the chromatographic fractions. ¹H NMR (400 MHz, CDCl₃): -0.33 - -0.38 (m, 1H), 0.42-0.47 (m, 1H), 0.63-0.67 (m, 1H), 0.75-0.82 (m, 1H), 0.89-1.02 (m, 1H), 0.98 (m, 6H), 1.24 (s, 12H).

### Preparation 12

### 5-[5-[(1S,2R)-2-Isopropylcyclopropyl]-6-methyl-pyridazin-3-yl]-2,4-dimethoxy-pyrimidine

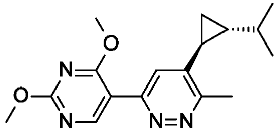

Mix 4-chloro-6-(2,4-dimethoxypyrimidin-5-yl)-3-methyl-pyridazine (19.1 g, 70.6 mmol), K₃PO₄ (45.9 g, 212 mmol), 1,4-dioxane (300 mL) and H₂O (75 mL) and degas the mixture with N₂ for 10 minutes. Add [1,1'-bis(diphenylphosphino)ferrocene]dichloro palladium(II) (7.98 g 10.6 mmol). Bubble with nitrogen for 2 additional minutes and add 2-[(1S,2S)-2-isopropylcyclopropyl]-4,4,5,5-tetramethyl-1,3,2-dioxaborolane (22.2 g, 106 mmol) in one portion. Stir the resulting mixture at 80 °C for 16 hours. Cool the reaction to RT, add H₂O, and extract with EtOAc. Separate the organic layer, dry over anhydrous MgSO₄, and concentrate under reduced pressure. Purify the resulting residue by chromatography over silica, eluting with 85% hexanes/EtOAc, to isolate title compound (21.5 g, 92%) as an amber oil, after solvent removal from the chromatographic fraction. The oil solidifies on standing at RT to an off-white solid.. ES/MS (m/z): 315 (M+1).

### Example 1

### 5-[5-[(1S,2R)-2-Isopropylcyclopropyl]-6-methyl-pyridazin-3-yl]-1H-pyrimidine-2,4-dione

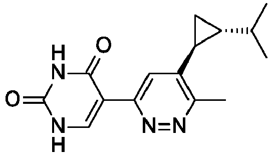

Dissolve trans-5-[5-[2-isopropylcyclopropyl]-6-methyl-pyridazin-3-yl]-2,4-dimethoxy-pyrimidine isomer 1 (628 mg, 2.00 mmol) in MeOH (3 mL). Add 1 M aqueous solution of HCl (5 mL) and heat to 70 °C for 3 hours. Cool to RT, load reaction mixture on MeOH-washed SCX column (20 g, Silicycle SILIABOND Tosic Acid), wash SCX column with MeOH (140 mL) and elute desired product with 2 M NH₃/MeOH (140 ml). Concentrate NH₃/MeOH fractions to obtain the title compound as a cream colored solid (546 mg, 95%). ES/MS (m/z): 287 (M+H). ¹H NMR (d₆-DMSO) δ: 0.99 (m, 9H), 1.24 (m, 1H), 1.81 (m, 1H), 2.72 (s, 3H), 7.67 (s, 1H), 8.23 (s, 1H), 11.43 (bs, 2H).

### X-Ray Powder Diffraction of Crystalline Forms

The free base compound is dissolved in methanol, stirred at 50C for 1 hour and allowed to cool to ambient temperature where it crystallizes from solution. The solids are then isolated by vacuum filtration and briefly dried under vacuum at 70C.

The XRD patterns of crystalline solids are obtained on a Bruker D4 Endeavor X-ray powder diffractometer, equipped with a CuKa source λ = 1.54060 Å) and a Vantec detector, operating at 35 kV and 50 mA. The sample is scanned between 4 and 40° in 2θ, with a step size of 0.008° in 2θ and a scan rate of 0.5 seconds/step, and with 0.6 mm divergence, 5.28 fixed anti-scatter, and 9.5 mm detector slits. The dry powder is packed on a quartz sample holder and a smooth surface is obtained using a glass slide. The crystal form diffraction patterns are collected at ambient temperature and relative humidity. It is well known in the crystallography art that, for any given crystal form, the relative intensities of the diffraction peaks may vary due to preferred orientation resulting from factors such as crystal morphology and habit. Where the effects of preferred orientation are present, peak intensities are altered, but the characteristic peak positions of the polymorph are unchanged. See, *e.g.* The United States Pharmacopeia #23, National Formulary #18, pages 1843-1844, 1995. Furthermore, it is also well known in the crystallography art that for any given crystal form the angular peak positions may vary slightly. For example, peak positions can shift due to a variation in the temperature or humidity at which a sample is analyzed, sample displacement, or the presence or absence of an internal standard. In the present case, a peak position variability of ± 0.2 in 2θ will take into account these potential variations without hindering the unequivocal identification of the indicated crystal form. Confirmation of a crystal form may be made based on any unique combination of distinguishing peaks (in units of ° 2θ), typically the more prominent peaks. The crystal form diffraction patterns, collected at ambient temperature and relative humidity, were adjusted based on NIST 675 standard peaks at 8.853 and 26.774 degrees 2-theta.

### ¹³C Solid State NMR (¹³C ssNMR)

¹³C Cross polarization/magic angle spinning NMR (solid-state NMR or ssNMR) spectra are obtained using a Bruker Avance II 400 MHz NMR spectrometer operating at a carbon frequency of 100.622 MHz and proton frequency of 400.131 MHz and equipped with a Bruker 4mm double resonance probe. TOSS sideband suppression is used along with cross polarization employing SPINAL64 decoupling and a RAMP 100 shaped H-nucleus CP pulse. Acquisition parameters are as follows: 90° proton r.f. pulse width of 2.6 µs, contact time is 2.0 ms, pulse repetition time of 3 s, MAS frequency of 10 kHz, spectral width of 30 kHz, acquisition time is 34 ms and the number of scans is 18,661. Chemical shifts are referenced to adamantane (δ = 29.5 ppm) in a separate experiment.

### Preparation 13

### Crystalline Form 1, 5-[5-[(1S,2R)-2-isopropylcyclopropyl]-6-methyl-pyridazin-3-yl]-1H-pyrimidine-2,4-dione (anhydrous)

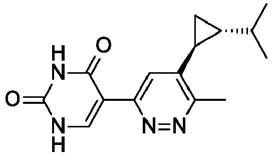

Stir a suspension of 5-[5-[(1S,2R)-2-isopropylcyclopropyl]-6-methyl-pyridazin-3-yl]-2,4-dimethoxy-pyrimidine (21.5 g, 65.6 mmol) and 1 M aqueous HCl solution (165 mL, 135 mmol) at 45 °C for 16 hours. Cool to RT and extract with MTBE. Discard the organic phase and add to the aqueous phase a 2 M aqueous solution of K₂HPO₄ until pH ~ 6 (approximately 150 mL). Stir the resulting mixture at RT for 16 hours. Filter and collect the resulting solid, washing with water and drying in a vacuum oven at 45 °C for 16 hours to yield the title compound (14.3 g, 76%) as a white solid. ES/MS (m/z): 287 (M+1).

A prepared sample of the 5-[5-[(1S,2R)-2-isopropylcyclopropyl]-6-methyl-pyridazin-3-yl]-1H-pyrimidine-2,4-dione anhydrous crystalline form (Form 1) is characterized by an XRD pattern using CuKa radiation as having diffraction peaks (2-theta values) as described in Table 1 below. Specifically, the pattern contains a peak at 4.7 ° in combination with one or more of the peaks selected from the group consisting of 11.7 °, 15.8°, and 16.0°; with a tolerance for the diffraction angles of 0.2 degrees.

**Table 1: X-ray powder diffraction peaks of the 5-[5-[(1S,2R)-2-isopropylcyclopropyl]-6-methyl-pyridazin-3-yl]-1H-pyrimidine-2,4-dione anhydrous crystalline form (Form 1).**

| **Peak** | **Angle** (2-Theta °) +/- 0.2° | **Relative Intensity (% of most intense peak)** |
|---|---|---|
| 1 | 4.7 | 100% |
| 2 | 11.7 | 6% |
| 3 | 15.8 | 6% |
| 4 | 16.0 | 10% |
| 5 | 26.8 | 5% |
| 6 | 27.2 | 4% |

Representative ¹³C ssNMR resonances for the 5-[5-[(1S,2R)-2-isopropylcyclopropyl]-6-methyl-pyridazin-3-yl]-1H-pyrimidine-2,4-dione anhydrous crystalline form (Form 1) include: 16.6, 18.3, 20.7, 22.1, 23.9, 25.4, 26.6, 27.6, 28.1, 28.9, 30.2, 31.2, 32.2, 38.6, 39.7, 41.2, 43.1, 109.3, 111.0, 112.0, 124.4, 128.2, 130.7, 145.1, 146.3, 150.1, 151.0, 153.4, 156.7, 157.6, 158.6, 159.3, 160.7, 163.4, 165.9, 167.0, 169.4, and 170.2 ppm (± 0.2 ppm respectively).

### Preparation 14

### Crystalline Form 2, 5-[5-[(1S,2R)-2-isopropylcyclopropyl]-6-methyl-pyridazin-3-yl]-1H-pyrimidine-2,4-dione (desolvate/anhydrous)

Add Crystalline Form 1, 5-[5-[(1S,2R)-2-isopropylcyclopropyl]-6-methyl-pyridazin-3-yl]-1H-pyrimidine-2,4-dione (15.4 kg, 53.8 mol) to water (770 L) in a 3500 L hastelloy reactor at RT. Stir the resulting suspension at RT for 19 hours. Filter and collect the resulting solid, washing with water and drying in a vacuum oven at 40 °C for 3 days to yield the title compound (13.8 kg, 89%) as a white solid. HLPC purity: 99.8%.

A family of related crystalline forms prepared from a sample of the 5-[5-[(1S,2R)-2-isopropylcyclopropyl]-6-methyl-pyridazin-3-yl]-1H-pyrimidine-2,4-dione desolvate crystalline form (Form 2) is observed. One family member is characterized by an XRD pattern using CuKa radiation as having diffraction peaks (2-theta values) as described in Table 2 below.

**Table 2: X-ray powder diffraction peaks of the 5-[5-[(1S,2R)-2-isopropylcyclopropyl]-6-methyl-pyridazin-3-yl]-1H-pyrimidine-2,4-dione desolvate crystalline form (Form 2).**

| **Peak** | **Angle** (2-Theta °) +/- 0.2° | **Relative Intensity (% of most intense peak)** |
|---|---|---|
| 1 | 4.3 | 100 |
| 2 | 6.4 | 56 |
| 3 | 9.6 | 13 |
| 4 | 11.0 | 11 |
| 5 | 11.6 | 36 |

Representative ¹³C ssNMR resonances for the 5-[5-[(1S,2R)-2-isopropylcyclopropyl]-6-methyl-pyridazin-3-yl]-1H-pyrimidine-2,4-dione desolvate (anhydrous) crystalline form (Form 2) include: 17.2, 18.9, 21.1, 22.5, 24.4, 26.7, 27.8, 28.7, 29.7, 39.7, 41.3, 45.5, 50.1, 110.7, 111.7, 112.4, 122.0, 125.0, 126.1, 129.1, 143.6, 144.8, 146.2, 147.2, 149.9, 151.1, 153.0, 155.5, 157.2, 159.0, 160.6, 161.4, 162.7, 164.8, 167.0, 168.3, and 170.4 ppm (± 0.2 ppm respectively).

The other family members are characterized by an XRD pattern using CuKa radiation as having a diffraction peak (2-theta values) at 4.3° in combination with one or more of the peaks of 4.7°, 6.4°, 9.3°, 11.0°, 11.6°, 18.4°, and 27.9°, with a tolerance for the diffraction angles of 0.2 degrees.

### Preparation 15

### Crystalline Form 3, 5-[5-[(1S,2R)-2-isopropylcyclopropyl]-6-methyl-pyridazin-3-yl]-1H-pyrimidine-2,4-dione (hemihydrate)

Add 5-[5-[(1S,2R)-2-isopropylcyclopropyl]-6-methyl-pyridazin-3-yl]-2,4-dimethoxy-pyrimidine (316 g, 0.92 mol) to a 1 M aqueous solution of HCl (2.6 L, 2.6 mol) in a 5 L flask at RT. Heat the resulting suspension to 45 °C and stir at this temperature for 16 hours. Cool to RT and add 2M aqueous K₂HPO₄ until pH ~ 6. Stir the resulting mixture at RT for 1 hour. Filter and collect the resulting solid, drying in a vacuum oven at 40 °C for 3 days to yield the title compound (250 g, 92%) as a white solid. HLPC purity: 99%.

A prepared sample of the 5-[5-[(1S,2R)-2-isopropylcyclopropyl]-6-methyl-pyridazin-3-yl]-1H-pyrimidine-2,4-dione hemihydrate crystalline form (Form 3) is characterized by an XRD pattern using CuKa radiation as having diffraction peaks (2-theta values) as described in Table 3 below. Specifically, the pattern contains a peak at 5.0° in combination with one or more of the peaks selected from the group consisting of 16.4 °, 17.8 °, and 26.6 °; with a tolerance for the diffraction angles of 0.2 degrees.

**Table 3: X-ray powder diffraction peaks of the 5-[5-[(1S,2R)-2-isopropylcyclopropyl]-6-methyl-pyridazin-3-yl]-1H-pyrimidine-2,4-dione hemihydrate crystalline form (Form 3).**

| **Peak** | **Angle** (2-Theta °) +/- 0.2° | **Relative Intensity (% of most intense peak)** |
|---|---|---|
| 1 | 5.0 | 100 |
| 2 | 16.4 | 15 |
| 3 | 17.8 | 10 |
| 4 | 26.6 | 23 |
| 5 | 28.8 | 8 |

Representative ¹³C ssNMR resonances for the 5-[5-[(1S,2R)-2-isopropylcyclopropyl]-6-methyl-pyridazin-3-yl]-1H-pyrimidine-2,4-dionehemihydrate crystalline form (Form 3) include: 16.5, 18.9, 19.6, 20.4, 22.6, 23.5, 24.4, 26.0, 27.4, 28.3, 30.0, 30.9, 34.6, 38.4, 40.5, 110.2, 110.8, 124.1, 126.8, 130.4, 131.2, 146.0, 147.1, 150.4, 151.2, 153.4, 157.1, 157.7, 158.3, 159.6, 163.1, 166.2, and 169.7 ppm (+ 0.2 ppm, respectively).

### Preparation 16

### Conversion of Crystalline Form 2 to Crystalline Form 1

Add Crystalline Form 2, 5-[5-[(1S,2R)-2-isopropylcyclopropyl]-6-methyl-pyridazin-3-yl]-1H-pyrimidine-2,4-dione (5.0 kg, 17.4 mol) to a mixture of THF (100 L) and water (10 L). Concentrate the resulting solution to 10-15 L under reduced pressure below 50 °C. Charge THF (5 L) and water (60 L), heat to 50-60 °C and stir for 2-4 hours. Cool to 20-25 °C, and stir for 1-2 hours. Filter and collect the resulting solid, washing with water (10 L) and drying to yield the title compound (4.2 kg, 84%) as an off-white solid. HLPC purity: 99.4%.

### Mass Spectroscopy for Adenosine and Adenosine Purification

An Agilent 300 RapidFire automated extraction system (Agilent, Santa Clara, CA) is used with three HPLC quaternary pumps, coupled to an Sciex 6500 triple quadrupole mass spectrometer (AB Sciex, Framingham, MA) with an electrospray ionization (ESI) interface source. Load a RapidFire Mass Spec system with reusable RapidFire HILIC (H1) solid-phase extraction (SPE) cartridge (G9203-80109).

Solvent A, used for sample loading and washing, is 50 mM ammonium formate, pH 4.0 containing 5% (v/v) ACN. Solvent B, used for sample elution, is 0.3% formic acid + 2% ammonium hydroxide in 70% ACN/30% MeOH. Samples are sequentially analyzed by aspirating 10 µL onto the collection loop under vacuum directly from multiwell plates. Load 10 µL of sample is onto the HILIC cartridge and wash, by quaternary pump 1, using solvent A at a flow rate of 1.25 mL/minute for 3000 ms. The retained analytes elute to the mass spectrometer by quaternary pump 3, using solvent B at a flow rate of 1.25 mL/minute for 3000 ms. The system is re-equilibrated by quaternary pump 1, using solvent A at a flow rate of 1.25 mL/min for 3000 ms.

Equip the triple quadrupole mass spectrometer with an electrospray ionization (ESI) source and monitor analytes using selected reaction monitoring (SRM) in positive mode (M + H)+. Monitor adenosine at *m*/*z* 268.05/136.0 and adenosine monophosphate at *m*/*z* 348.1/136.0. Calculate area ratio values for adenosine and adenosine monophosphate using 13C5 Adenosine and 15N5 AMP as internal standards, respectively.

### Human CD73 Biochemical Assay

The purpose of this assay is to identify and characterize 5-[5-[(1S,2R)-2-isopropylcyclopropyl]-6-methyl-pyridazin-3-yl]-1H-pyrimidine-2,4-dione against CD73 enzyme activity. Add the reaction mixtures (20 µL) containing 2 µM adenonsine monophosphate (Sigma #01930), 10 mM Tris pH 7.5, 100 mM NaCl, 0.01% BSA, 0.2 mM Octyl glucoside, and 50 pM CD73 protein to a 384 well plate (Nunc #264573). After 30 minutes incubation at RT, terminate the reaction by adding 20 µL stop solution containing 2% formic acid and 10 µM ¹³C5-adenosine (ribose labeled with ¹³C5) (Cambridge Isotope Laboratories - #CLM-3678-0) followed by addition of 40 µL dH₂O. Adenosine-and adenosine ribose-¹³C5 (internal standard) levels are determined utilizing mass spectrometry as described above . Use signal ratio (adenosine peak integration / adenosine internal standard peak integration) to quantitate each reaction. Calculate percent inhibition by using the equation {% inhibition = 100 × [1-(X-MIN)/(MAX-MIN)]} where X equals the well signal ratio, MAX equals median signal ratio of DMSO control and MIN equals signal ratio of enzyme activity in the presence of >10X IC₅₀ of a known competitive inhibitor. For screening purposes, test each compound at 50 µM in 1% DMSO. Determine IC₅₀ of 5-[5-[(1S,2R)-2-isopropylcyclopropyl]-6-methyl-pyridazin-3-yl]-1H-pyrimidine-2,4-dione by testing each compound at 10 concentrations from 0.0025 to 50 µM (using a 1:3 dilution scheme).

The IC₅₀ for 5-[5-[(1S,2R)-2-isopropylcyclopropyl]-6-methyl-pyridazin-3-yl]-1H-pyrimidine-2,4-dione is 0.028 µM.

### Mouse CD73 Mechanism Assay

The purpose of this assay is to assess 5-[5-[(1S,2R)-2-isopropylcyclopropyl]-6-methyl-pyridazin-3-yl]-1H-pyrimidine-2,4-dione with regard to its inhibition of mouse CD73 enzyme activity. This assay is carried out as described above for human CD73 biochemical assay, except that 3 µM AMP and 50 pM mouse CD73 enzyme are used.

The IC₅₀ for 5-[5-[(1S,2R)-2-isopropylcyclopropyl]-6-methyl-pyridazin-3-yl]-1H-pyrimidine-2,4-dione is 0.175 µM.

### Calu6 Human Cell Assay

The purpose of this assay is to test 5-[5-[(1S,2R)-2-isopropylcyclopropyl]-6-methyl-pyridazin-3-yl]-1H-pyrimidine-2,4-dione against CD73 in a cell-based assay. Calu6 cells (1500 cells / well) are grown in a 96 well Poly-D-Lysine coated plate (BD #356640) containing 100 µL of media (MEM (Gibco #11095-072) + 1% Sodium Pyruvate (Gibco #11360-070) + 1% NEAA (Gibco #11140-050) + 10% FBS (Hyclone #SH30071). Plates are incubated at RT for 30 minutes, followed by incubation overnight at 37 °C / 5% CO₂. Cells are washed twice with assay buffer (10 mM Tris-HCl pH 7.2, 10 mM D-Glucose, 1 mM KCl, 125 mM NaCl, 2 mM MgCl₂)(90 µL/well). Then, 90 µL assay buffer is added to each well followed with addition of 10 µL per well of AMP and compound premix (50 µM AMP, varying concentrations of 5-[5-[(1S,2R)-2-isopropylcyclopropyl]-6-methyl-pyridazin-3-yl]-1H-pyrimidine-2,4-dione in 1% DMSO). Plates are incubated at room temperature for 60 minutes. Then, 10 µL supernatant per well is removed and added to a new plate followed by addition of 20 µL stop solution (2% formic acid, 1.2 µM Adenosine Ribose- ¹³C5 (Cambridge Isotope Laboratories - #CLM-3678-0) and 90 µL ddH2O for mass spectroscopy analysis. Adenosine- and Adenosine Ribose- ¹³C5 (internal standard) levels are determined by utilizing mass spectrometry (Agilent RapidFire) as described above for human CD73 biochemical assay. Percent inhibition is also calculated as described above.

The IC₅₀ for 5-[5-[(1S,2R)-2-isopropylcyclopropyl]-6-methyl-pyridazin-3-yl]-1H-pyrimidine-2,4-dione is 0.0073 uM (the compound of Example 2).

### Ex Vivo Tarset inhibition Assay

The purpose of this assay is to test 5-[5-[(1S,2R)-2-isopropylcyclopropyl]-6-methyl-pyridazin-3-yl]-1H-pyrimidine-2,4-dione against murine CD73 in mouse blood in an *ex vivo* based assay. The animals (6/group) are dosed orally with each compound formulated in 20% HPBCD (2-hydroxypropyl-β-cyclodextrin), pH 2 after tumors reach to approximately 400 mm³. After treatment, blood is collected into heparin tubes and used for ex vivo analysis of conversion of ¹³C10-¹⁵N5-AMP to labeled-adenosine, inosine, and hypoxanthine as described for ex vivo assay using whole blood collected from animals subjected to compound treatment via oral dosing.

5-[5-[(1S,2R)-2-isopropylcyclopropyl]-6-methyl-pyridazin-3-yl]-1H-pyrimidine-2,4-dione inhibits the conversion of AMP to adenosine, inosine, and hypoxanthine in mouse whole blood from animals treated with different doses of the compound, as shown in Table 4.

| Table 4. Inhibition of AMP conversion to adenosine | | |
|---|---|---|
| Group | Inhibition (%) | p-value |
| Vehicle | 0.0 | 1.000 |
| 1 mg/kg | -10.6 | 0.9139 |
| 2.5 mg/kg | 25.2 | 0.162 |
| 6.4 mg/kg | 37.0 | 0.0127* |
| 16 mg/kg | 56.5 | <0.0001 * |
| 40 mg/kg | 88.6 | <0.0001 * |
| 100 mg/kg | 94.3 | <0.0001 * |

| | | |
|---|---|---|
| *: statistical significance. | | |

The IC₅₀ for 5-[5-[(1S,2R)-2-isopropylcyclopropyl]-6-methyl-pyridazin-3-yl]-1H-pyrimidine-2,4-dione is 0.0073 uM.

### Calu6 tumor based in vivo target inhibition of human CD73 assay

The purpose of this assay is to test 5-[5-[(1S,2R)-2-isopropylcyclopropyl]-6-methyl-pyridazin-3-yl]-1H-pyrimidine-2,4-dione against human CD73 in xenograft tumors derived from human cancer Calu6 cells in an in vivo target inhibition assay. Calu6 cells (ATCC) are grown in HBSS medium supplemented with 10% fetal bovine serum. Harvest sub-confluent cells with trypsin and rinse twice with growth medium lacking serum. Initiate the growth of subcutaneous tumors by injecting 5 × 10⁶ in a 1:1 mixture of HBSS and MATRIGEL^{®} (BD Biosciences, Franklin Lakes, NJ) in the rear flank of nude mice (The Harlon Laboratory). When the mean tumor volume reaches approximately 400-500 mm³, randomize the animals by tumor size and body weight and place into their respective treatment groups as indicated. After treatment, tumor samples (50-80 mg each) are collected and processed in 1 mL of ice cold extraction buffer containing internal standards as described below.

A foil strip and liquid N₂ are added to a the mortar to pre-chill. Tumor tissue is dropped onto the foil strip, and liquid N₂ is added. Another foil strip is placed on top of the tumor tissue and is hammered with the pestle until the tumors are thoroughly grounded. 50 to 100 mg of tumor tissue is placed into tubes (Fishers Scientific, cat# 02-681-302) and placed on dry ice. One metal bead (Qiagen Cat. No. 69989) and 1 ml of 80% of methanol containing the internal standards of ¹³C₅-Adenosine, ¹³C₅-AMP, ¹⁵N₅-GTP, ¹⁵N₄-inosine 5'-monophosphate, and ¹³C-¹⁵N-Hypoxanthine (Cambridge Isotope Lab and Cayman Chemical) are added to the tubes, and samples are stored at -80°C until use for LC/MS analysis.

Blood is also collected into heparin tubes and used for ex vivo analysis of conversion of ¹³C₅-¹⁵N5-AMP to labeled-adenosine, inosine, and hypoxanthine as described for ex vivo assay using whole blood collected from animals subjected to compound treatment via oral dosing.

As shown in Table 5, 5-[5-[(1S,2R)-2-isopropylcyclopropyl]-6-methyl-pyridazin-3-yl]-1H-pyrimidine-2,4-dione inhibits the conversion of AMP to adenosine in Calu6 tumors treated with different doses of the compound.

| Table 5. Inhibition of AMP conversion to adenosine | | |
|---|---|---|
| Compound Treatment Group (mg/kg) | Inhibition (%) | p-value |
| 0.0 | 0 | 1.000 |
| 1.0 | 39.1 | 0.0057* |
| 2.5 | 68.5 | <0.0001* |
| 6.4 | 64.3 | <0.0001* |
| 16.0 | 77.5 | <0.0001* |
| 40.0 | 82.1 | <0.0001* |
| 100.0 | 89.3 | <0.0001* |

| | | |
|---|---|---|
| *: statistical significance. | | |

### LC/MS based assay for measuring CD73 activity in human serum

Fresh normal human blood is centrifuged at 1,500 *g* for 15 minutes at room temperature, and the upper fraction containing serum is collected. Serum (25ul/well) collected is transferred to a 96-deep well plate (DWP, Analytical Sales & Services Inc. Cat# 968820) containing various concentrations of 5-[5-[(1S,2R)-2-isopropylcyclopropyl]-6-methyl-pyridazin-3-yl]-1H-pyrimidine-2,4-dione and a fixed concentration of levamisole (1,500 uM). After incubation on ice for 60 min, ¹³C5-¹⁵N5-AMP (50 uM) is added to each well in the plate, and the plate is incubated at room temperature for 15 min. The plate is then placed on dry ice with addition of 200 uL/well of 17.3 TCA followed by shaking at 26 fps for 3 min with a plate-shaking machine (Qiagen). The plate is then centrifuged at 2940 g for 20 min at 4 °C. After centrifugation, 100ul/well of supernatant from each well is transferred to a new 96 deep well plate and mixed with 18.4ul/well of 2.5M Na₂CO₃ on ice followed by addition of 200 ul of extraction solution containing internal standard (IS:¹³C5-AMP, ¹³C5-adenosine, ¹³C5-hypoxanthine, and ¹⁵N4-inosine). After further centrifugation at 2940 g for 20 min at 4°C, 200 ul/well of the supernatant is used for analysis of ¹³C10-¹⁵N5-adenosine, ¹³C10-¹⁵N5-inosine and ¹⁵N5-hypoxanthine by LC/MS as described above. For EC50 calculations, the concentrations of 5-[5-[(1S,2R)-2-isopropylcyclopropyl]-6-methyl-pyridazin-3-yl]-1H-pyrimidine-2,4-dione in the serum are corrected with the fraction unbound (%) derived from in silico models or experimentally.

Table 6 contains data for inhibition of CD73 activity in human serum by 5-[5-[(1S,2R)-2-isopropylcyclopropyl]-6-methyl-pyridazin-3-yl]-1H-pyrimidine-2,4-dione.

| Table 6 | |
|---|---|
| 5-[5-[(1S,2R)-2-isopropylcyclopropyl]-6-methyl-pyridazin-3-yl]-1H-pyrimidine-2,4-dione (uM) | Inhibition (%) |
| 4.6800 | 76.9 |
| 1.5600 | 71.1 |
| 0.5200 | 56.8 |
| 0.1733 | 39.6 |
| 0.0578 | 25.4 |
| 0.0193 | 15.4 |
| 0.0064 | 8.4 |
| 0.0021 | 3.7 |
| 0.0007 | 0.7 |
| 0 | 0.0 |

### In Vivo Models

If desired, pre-clinical modeling of the CD73-inhibitory effect of 5-[5-[(1S,2R)-2-isopropylcyclopropyl]-6-methyl-pyridazin-3-yl]-1H-pyrimidine-2,4-dione, or a pharmaceutically acceptable salt thereof, can be performed, for example, according to methods set forth in the art, for example, in Rongvaux A, et al., Annual Rev. Immunology 2013; 31: 635-74, and in literature cited therein; Sanmamed MF, et al., Annals of Oncology 2016; 27: 1190-1198, and in literature cited therein.

## Claims

1. A crystalline form of the compound of the formula: 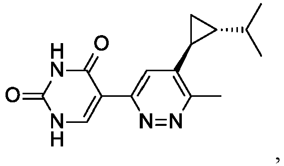 which is **characterized by** at least one of:
(a) a peak in the X-ray diffraction pattern, at a diffraction angle 2-theta of 4.3°, in combination with one or more of the peaks at 6.4°, 9.6°, 11.0° and 11.6°; with a tolerance for the diffraction angles of 0.2 degrees; and
(b) a ¹³C solid state NMR spectrum which comprises peaks referenced to the highfield resonance of adamantane (δ = 29.5 ppm) at: 17.2, 28.7, 122.0, 126.1, 143.6 and 155 ppm (+ 0.2 ppm, respectively).

2. A composition comprising the crystalline form according to Claim 1.

3. A pharmaceutical composition comprising the crystalline form according to Claim 1, and one or more pharmaceutically acceptable carriers, diluents, or excipients.

4. The crystalline form according to Claim 1, which is at least 95% free of contaminants.

5. A hemihydrate crystalline form of the compound of the formula:

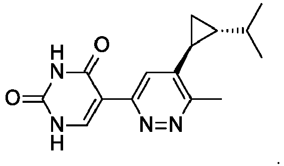

6. The hemihydrate crystalline form according to Claim 5, which is **characterized by** at least one of:
(a) a peak in the X-ray diffraction pattern, at a diffraction angle 2-theta of 5.0°, in combination with one or more of the peaks at 16.4°, 17.8 and 26.6°; with a tolerance for the diffraction angles of 0.2 degrees; and
(b) a ¹³C solid state NMR spectrum which comprises peaks referenced to the highfield resonance of adamantane (δ = 29.5 ppm) at: 23.5, 34.6, 110.2, 124.1, 126.8 and 166.2 ppm (± 0.2 ppm, respectively).

7. A composition comprising the hemihydrate crystalline form according to Claim 5 or Claim 6.

8. A pharmaceutical composition comprising the hemihydrate crystalline form according to Claim 5 or Claim 6, and one or more pharmaceutically acceptable carriers, diluents, or excipients.

9. The hemihydrate crystalline form according to Claim 5 or Claim 6, wherein the crystalline form is at least 95% free of contaminants.

10. A composition comprising 500 g of an anhydrous crystalline form of the compound of the formula:

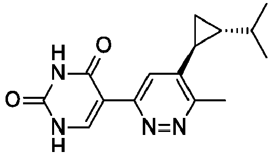

11. The composition according to Claim 10, wherein the anhydrous crystalline form is **characterized by** at least one of:
(a) a peak in the X-ray diffraction pattern, at a diffraction angle 2-theta of 4.7°, in combination with one or more of the peaks at 11.7°, 15.8 °, 16.0°, 26.8°, and 27.2°; with a tolerance for the diffraction angles of 0.2 degrees; and
(b) a ¹³C solid state NMR spectrum which comprises peaks referenced to the highfield resonance of adamantane (δ = 29.5 ppm) at: 25.4, 28.9, 43.1, 109.3, 124.4, 128.2 and 160.7 ppm (+ 0.2 ppm, respectively).

12. A pharmaceutical composition comprising the composition according to Claim 10 or Claim 11, and one or more pharmaceutically acceptable carriers, diluents, or excipients.

13. The composition according to Claim 2, Claim 7, Claim 10 or Claim 11, wherein the composition is a granulate composition.

14. A manufacturing container comprising at least 1 kg of the composition according to Claim 2, Claim 7, Claim 10 or Claim 11.

## Patentansprüche

1. Kristalline Form der Verbindung der folgenden Formel: 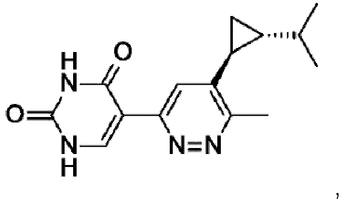 welche durch mindestens eines der folgenden gekennzeichnet ist:
(a) einen Peak im Röntgenbeugungsmuster bei einem Beugungswinkel 2-Theta von 4,3°, in Kombination mit einem oder mehreren der Peaks bei 6,4°, 9,6°, 11,0° und 11,6°; mit einer Toleranz für die Beugungswinkel von 0,2 Grad;
und
(b) ein ¹³C-Festkörper-NMR-Spektrum, das Peaks umfasst, die auf die Hochfeldresonanz von Adamantan (δ = 29,5 ppm) bezogen sind bei: 17,2, 28,7, 122,0, 126,1, 143,6 und 155 ppm (jeweils ± 0,2 ppm).

2. Zusammensetzung, welche die kristalline Form nach Anspruch 1 aufweist.

3. Pharmazeutische Zusammensetzung, welche die kristalline Form nach Anspruch 1 und einen oder mehrere pharmazeutisch verträgliche Träger, Verdünnungsmittel oder Hilfsstoffe aufweist.

4. Kristalline Form nach Anspruch 1, welche zu mindestens 95% frei von Verunreinigungen ist.

5. Kristalline Form des Halbhydrats der Verbindung der folgenden Formel:

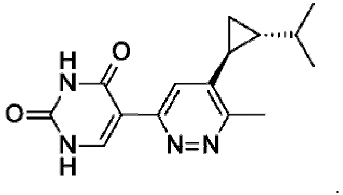

6. Kristalline Form des Halbhydrats nach Anspruch 5, welche durch mindestens eines der folgenden gekennzeichnet ist:
(a) einen Peak im Röntgenbeugungsmuster bei einem Beugungswinkel 2-Theta von 5,0°, in Kombination mit einem oder mehreren der Peaks bei 16,4°, 17,8° und 26,6°; mit einer Toleranz für die Beugungswinkel von 0,2 Grad; und
(b) ein ¹³C-Festkörper-NMR-Spektrum, welches Peaks umfasst, die auf die Hochfeldresonanz von Adamantan (δ = 29,5 ppm) bezogen sind, bei: 23,5, 34,6, 110,2, 124,1, 126,8 und 166,2 ppm (jeweils ± 0,2 ppm).

7. Zusammensetzung, welche die kristalline Form des Halbhydrats nach Anspruch 5 oder Anspruch 6 aufweist.

8. Pharmazeutische Zusammensetzung, welche die kristalline Form des Halbhydrats nach Anspruch 5 oder Anspruch 6 und einen oder mehrere pharmazeutisch verträgliche Träger, Verdünnungsmittel oder Hilfsstoffe aufweist.

9. Kristalline Form des Halbhydrats nach Anspruch 5 oder Anspruch 6, wobei die kristalline Form zu mindestens 95 % frei von Verunreinigungen ist.

10. Zusammensetzung, die 500 g einer wasserfreien kristallinen Form der Verbindung der folgenden Formel enthält:

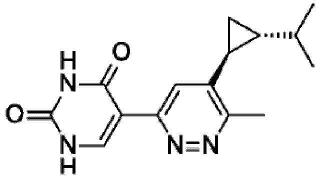

11. Zusammensetzung nach Anspruch 10, wobei die wasserfreie kristalline Form durch mindestens eines der folgenden gekennzeichnet ist:
(a) einen Peak im Röntgenbeugungsmuster bei einem Beugungswinkel 2-Theta von 4,7°, in Kombination mit einem oder mehreren der Peaks bei 11,7°, 15,8°, 16,0°, 26,8° und 27,2°; mit einer Toleranz für die Beugungswinkel von 0,2 Grad; und
(b) ein ¹³C-Festkörper-NMR-Spektrum, welches Peaks umfasst, die auf die Hochfeldresonanz von Adamantan (δ = 29,5 ppm) bezogen sind, bei: 25,4, 28,9, 43,1, 109,3, 124,4, 128,2 und 160,7 ppm (jeweils ± 0,2 ppm).

12. Pharmazeutische Zusammensetzung, umfassend die Zusammensetzung nach Anspruch 10 oder Anspruch 11 und einen oder mehrere pharmazeutisch verträgliche Träger, Verdünnungsmittel oder Hilfsstoffe.

13. Zusammensetzung nach Anspruch 2, Anspruch 7, Anspruch 10 oder Anspruch 11, wobei die Zusammensetzung eine Granulat-Zusammensetzung ist.

14. Herstellungsbehälter, der mindestens 1 kg der Zusammensetzung nach Anspruch 2, Anspruch 7, Anspruch 10 oder Anspruch 11 enthält.

## Revendications

1. Forme cristalline du composé de formule : 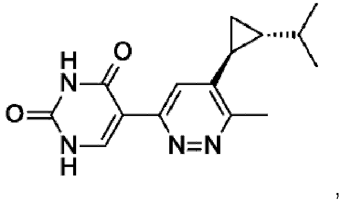 qui est **caractérisée par** au moins l'un de :
(a) un pic dans le diagramme de diffraction des rayons X, à un angle de diffraction 2-thêta de 4,3 °, en combinaison avec un ou plusieurs des pics à 6,4 °, 9,6 °, 11,0 ° et 11,6 °; avec une tolérance pour les angles de diffraction de 0,2 degré ;
et
(b) un spectre RMN à l'état solide du ¹³C qui comprend des pics référencés à la résonance de champ élevé de l'adamantane (δ = 29,5 ppm) à : 17,2, 28,7, 122,0, 126,1, 143,6 et 155 ppm (± 0,2 ppm, respectivement).

2. Composition comprenant la forme cristalline selon la revendication 1.

3. Composition pharmaceutique comprenant la forme cristalline selon la revendication 1, et un ou plusieurs supports, diluants ou excipients pharmaceutiquement acceptables.

4. Forme cristalline selon la revendication 1, qui est au moins à 95 % exempte de contaminants.

5. Forme cristalline hémihydratée du composé de formule :

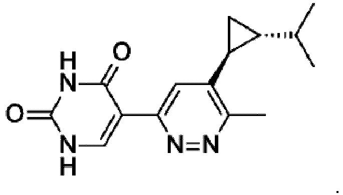

6. Forme cristalline hémihydratée selon la revendication 5, qui est **caractérisée par** au moins l'un de :
(a) un pic dans le diagramme de diffraction des rayons X, à un angle de diffraction 2-thêta de 5,0 °, en combinaison avec un ou plusieurs des pics à 16,4 °, 17,8 ° et 26,6 °; avec une tolérance pour les angles de diffraction de 0,2 degré ; et
(b) un spectre RMN à l'état solide du ¹³C qui comprend des pics référencés à la résonance de champ élevé de l'adamantane (δ = 29,5 ppm) à : 23,5, 34,6, 110,2, 124,1, 126,8 et 166,2 ppm (± 0,2 ppm, respectivement).

7. Composition comprenant la forme cristalline hémihydratée selon la revendication 5 ou la revendication 6.

8. Composition pharmaceutique comprenant la forme cristalline hémihydratée selon la revendication 5 ou la revendication 6, et un ou plusieurs supports, diluants ou excipients pharmaceutiquement acceptables.

9. Forme cristalline hémihydratée selon la revendication 5 ou la revendication 6, dans laquelle la forme cristalline est exempte d'au moins 95 % de contaminants.

10. Composition comprenant 500 g d'une forme cristalline anhydre du composé de formule :

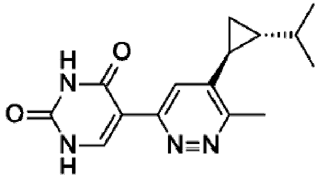

11. Composition selon la revendication 10, dans laquelle la forme cristalline anhydre est **caractérisée par** au moins l'un de :
(a) un pic dans le diagramme de diffraction des rayons X, à un angle de diffraction 2-thêta de 4,7 °, en combinaison avec un ou plusieurs des pics à 11,7 °, 15,8 °, 16,0 °, 26,8 ° et 27,2 ° ; avec une tolérance pour les angles de diffraction de 0,2 degré ; et
(b) un spectre RMN à l'état solide du ¹³C qui comprend des pics référencés à la résonance de champ élevé de l'adamantane (δ = 29,5 ppm) à : 25,4, 28,9, 43,1, 109,3, 124,4, 128,2 et 160,7 ppm (± 0,2 ppm, respectivement).

12. Composition pharmaceutique comprenant la composition selon la revendication 10 ou la revendication 11, et un ou plusieurs supports, diluants ou excipients pharmaceutiquement acceptables.

13. Composition selon la revendication 2, la revendication 7, la revendication 10 ou la revendication 11, dans laquelle la composition est une composition granulée.

14. Récipient de fabrication comprenant au moins 1 kg de la composition selon la revendication 2, la revendication 7, la revendication 10 ou la revendication 11.
